# EUROPÄISCHE PATENTANMELDUNG

(11) **EP 0 904 803 A2**
(43) Veröffentlichungstag der Anmeldung: **31.03.1999**
(21) Anmeldenummer: 98118037.5
(22) Anmeldetag: 23.09.1998
(51) Int. Cl.: A61N 5/06

(54) **Vorrichtung zur kohärenten Verstärkung elektromagnetischer Schwingungen**

(30) Priorität: 25.09.1997 DE 19742299
(71) Anmelder: Ruschke, Thomas, 24340 Eckernförde (DE)
(72) Erfinder: Ruschke, Thomas, 24340 Eckernförde (DE)
(74) Vertreter: Sartorius, Peter, Dipl.-Ing.

(57) **Zusammenfassung**

**Die Erfindung bezieht sich auf** eine Vorrichtung 4 zur kohärenten Verstärkung elektromagnetischer Schwingungen durch induzierte Emission mittels eines Festkörpers, mit Neodym dotierte Kristalle oder Gläser, Halbleiter-Dioden oder Flüssigkeiten oder Gase, wobei die aus der Vorrichtung austretenden elektromagnetischen Schwingungen über ein Kabel oder Glasfaserkabel 2 zur Beeinflussung auf ein biologisches System, insbesondere auf einen menschlichen oder Tierkörper 11, geleitet werden. Das Kabel 2 weist eine Auslaßöffnung 7 auf, und die aus dem Kabel 2 austretenden Schwingungen oder Strahlen werden über einen Festkörper, ein Gemisch oder eine flüssige oder gasförmige Lösung 8 zur Beeinflussung auf ein biologisches System 11 geleitet, wobei der Festkörper, das Gemisch, die flüssigen oder die gasförmigen Lösungen mineralische, pflanzliche, tierische, menschliche Extrakte bzw. Produkte oder Giftstoffe als Beimischungen enthalten.

## Beschreibung

**Die Erfindung bezieht sich auf** eine Vorrichtung zur kohärenten Verstärkung elektromagnetischer Schwingungen durch induzierte Emission mittels eines Festkörpers, mit Neodym dotierte Kristalle oder Gläser, Halbleiter-Dioden oder Flüssigkeiten oder Gase, wobei die aus der Vorrichtung austretenden elektromagnetischen Schwingungen über ein Kabel oder Glasfaserkabel zur Beeinflussung auf ein biologisches System, insbesondere auf einen menschlichen Körper oder einen Tierkörper, geleitet werden.

**Es sind bereits** Vorrichtungen zur kohärenten Verstärkung elektromagnetischer Schwingungen durch induzierte Emission mittels eines Festkörpers bekannt, die in der Medizin eingesetzt werden. Bei den bekannten Lasern besteht ein Interesse an der hohen Impulsleistung und Bündelung der Laserstrahlen. Zur Zeit werden beim Menschen nur Retina-Ablösungen und spezielle Arten des Gehirnkrebses mit Laserstrahlen behandelt. Die Behandlung der Netzhautablösung ist einfach, und der Patient muß nicht in Narkose gehalten werden. Nach den bisher durchgeführten Operationen tritt bei dem operierten Patienten kein Schmerz auf, und der Patient fühlt sich lediglich eine Zeitlang geblendet, was auf die Wirkung des Laserlichts und auf chemische Vorgänge auf der Retina oder im Sehnerv zurückgeht. Bei der Krebsbehandlung im Gehirn mittels Laserstrahlen wird die ausgewählte Stelle vom Laserstrahl getroffen und eine Verdampfung des Gewebes herbeigeführt.

**Demgegenüber liegt der Erfindung die Aufgabe zugrunde**, die Vorrichtung zur kohärenten Verstärkung elektromagnetischer Schwingungen durch induzierte Emission gegenüber den bekannten Lasereinrichtungen zu verbessern und ein biologisches System positiv zu beeinflussen und/oder die Heilbehandlung, beispielsweise die Behandlung von Wunden, Brandwunden, Knochenbrüchen, Tumoren, Prellungen oder Blutergüssen, wesentlich zu beschleunigen.

**Gelöst wird die Aufgabe erfindungsgemäß dadurch**, daß das Kabel eine Auslaßöffnung aufweist und die aus dem Kabel austretenden Schwingungen oder Strahlen über einen Festkörper, ein Gemisch oder eine flüssige oder gasförmige Lösung zur Beeinflussung auf ein biologisches System geleitet werden, wobei der Festkörper, das Gemisch, die flüssigen oder die gasförmigen Lösungen mineralische, pflanzliche, tierische, menschliche Extrakte bzw. Produkte oder Giftstoffe als Beimischungen enthalten.

Entgegen der bisherigen Anwendung der Lasertechnik in der technischen oder thermischen Wirkung, beispielsweise beim Zertrümmern, Schneiden, Schweißen, Verdampfen, können mittels der erfindungsgemäßen Vorrichtung und durch die Wechselwirkung zwischen den Beimischungen und den elektromagnetischen Strahlen im sichtbaren Bereich spezifische Wirksamkeiten zur Entfaltung gebracht werden, und dadurch können diese Beimischungen im heilenden Sinne wirksam genutzt werden. Durch die Wechselwirkung zwischen den Lichtstrahlen und den in den Festkörpern oder Mischungen enthaltenen Substanzen wird eine wirksame Heilung in kurzer Zeit auf optimale Weise bewirkt. Mittels des Laserstrahls wird dem biologischen System die notwendige Energie in Verbindung mit den substanzspezifischen Informationen unter Ausnutzung des Ramann-Effekts zugeführt. Im vorliegenden Fall wurde beispielsweise eine Wunde zuerst im medizinisch klassischen Sinne versorgt und mit Antibiotika-Anlagen behandelt. Die Wunde hatte zu Beginn der Behandlung eine Länge von 18 cm und in der Mitte eine Breite von 2,5 cm. Die Wunde zeigte rohes Fleisch. Durch die Laserbestrahlung in Verbindung mit der in den Laserstrahl eingebrachten Substanz in einer Konzentration von 100 Vol.-% bis 10 x 10⁻²⁴ Vol.-% konnte eine optimale Heilung in kürzester Zeit, d. h. in drei Wochen, herbeigeführt werden. Dies wird in vorteilhafter Weise auch dadurch erreicht, daß der über die Substanz geführte Laserstrahl über das austretende Licht eine Information mitbekommt, die optisch dem Fingerabdruck der eingebrachten Substanz entspricht. Der austretende Laserstrahl wird in jedem Fall über den als optische Einrichtung wirkenden Festkörper bzw. die flüssige oder gasförmige Lösung mit der Beimischung geführt und auf die Wunde eingestrahlt. Die Behandlungszeit bei einer Wunde von 25 cm² kann 30 Minuten betragen. Eine derartige Behandlung über eine Woche, täglich fortgeführt, ergibt eine starke Heilreaktion, wobei die Wundgröße schließlich auf 4 cm² zusammenschrumpft und nach ca. zwei Wochen eine kreisrunde Borke aufweist. Wird ein derartiger Behandlungsprozeß über eine Zeit von drei Wochen geführt, so kann eine Wunde von ca. 25 cm² vollständig abheilen.

Dazu ist es vorteilhaft, daß das Kabel eine Einlaßöffnung und die Auslaßöffnung aufweist, wobei die Auslaßöffnung in die Einlaßöffnung einer Behandlungssonde einmündet und zwischen den Ein- und Auslaßöffnungen der Festkörper, das Gemisch oder die gasförmige oder flüssige Lösung vorgesehen bzw. einbringbar ist.

Eine zusätzliche Möglichkeit ist gemäß einer Weiterbildung der erfindungsgemäßen Vorrichtung, daß die Behandlungssonde oder die Aufnahmevorrichtung eine Längsöffnung und eine die Längsöffnung in einem Winkel schneidende Queröffnung aufweist, in die der Festkörper, das Gemisch oder die gasförmige oder flüssige Lösung mittel- oder unmittelbar einbringbar ist.

In weiterer Ausgestaltung der Erfindung ist es vorteilhaft, daß die Queröffnung im Strahleingang der elektromagnetischen Schwingungen bzw. des Laserstrahls liegt und zur Aufnahme eines Behälters dient, der zur Aufnahme des Festkörpers, des Gemischs oder der gasförmigen oder flüssigen Lösung dient.

Gemäß einer bevorzugten Ausführungsform der erfindungsgemäßen Lösung ist schließlich vorgesehen, daß der Behälter zur Aufnahme des Festkörpers, der gasförmigen oder flüssigen Substanz oder des Gemischs aus einem Glas oder einem Quarzglas besteht.

Von besonderer Bedeutung ist für die vorliegende Erfindung, daß der Behälter in die Queröffnung einsetzbar und festklemmbar und/oder arretierbar ist.

Im Zusammenhang mit der erfindungsgemäßen Ausbildung und Anordnung ist es von Vorteil, daß der Behälter mit einer Verschlußeinrichtung versehen ist.

Vorteilhaft ist es ferner, daß die Vorrichtung oder die Laservorrichtung als Argon-Ion-Laser und/oder als Argon-Ion-Multiline-Laser für Frequenzen von 5 x 10¹⁴ Hz bis 5 x 10¹⁵ Hz ausgelegt ist.

Außerdem ist es vorteilhaft, daß die in den Behälter eingegebene Beimischung in eine Trägerlösung aus Wasser und Aethanol eingegeben ist, wobei die Beimischung der Substanzen in einer Konzentration von 90 Vol.-% bis 10 x 10⁻²⁴ Vol.-% vorliegt.

Hierzu ist es vorteilhaft, daß die Laservorrichtung eine Leistungsaufnahme zwischen 30 mW und 100 mW oder 40 mW und 70 mW oder 45 mW und 65 mW oder 45 mW und 55 mW, insbesondere 50 mW, hat. Werden sehr große Körperflächen behandelt, so kann die Leistung entsprechend der Fläche erhöht werden.

Ferner ist es vorteilhaft, daß der Laser als Argon-Ion-Krypton-Mischgas-Laser ausgelegt ist.

Eine zusätzliche Möglichkeit ist gemäß einer Weiterbildung der erfindungsgemäßen Vorrichtung, daß der Laser als He-Ne-Laser ausgelegt ist.

In weiterer Ausgestaltung der Erfindung ist es vorteilhaft, daß der Laser als He-Cd-Laser ausgebildet ist.

Ferner ist es vorteilhaft, daß der Laser als Multiline-Singleline-, Multimod- oder als durchstimmbarer Laser ausgebildet ist.

In weiterer Ausgestaltung der Erfindung ist es vorteilhaft, daß der Laser als Dioden-Laser für Frequenzen von 5 x 10¹⁴ Hz bis 10¹⁵ Hz ausgelegt ist.

Eine zusätzliche Möglichkeit gemäß einer Weiterbildung der erfindungsgemäßen Vorrichtung besteht darin, daß der Laser als Kristall-Laser bzw. als Neodym-Yak-Laser ausgelegt ist.

Von Vorteil ist es ferner, daß in die Aufnahmevorrichtung zur Behandlung von Prellungen ein Festkörper oder gasförmiges oder flüssiges Gemisch einbringbar ist, das in der Konzentration von 10 x 10⁻³ Vol.-% bis 10 x 10⁻⁹ Vol.-% vorliegt.

Eine wesentliche vorteilhafte Ausführungsform erreicht man dadurch, daß in die Aufnahmevorrichtung zur Behandlung von Knochenbrüchen ein Festkörper oder gasförmiges oder flüssiges Gemisch einbringbar ist, das in der Konzentration von 10 x 10⁻³ Vol.-% bis 10 x 10⁻¹² Vol.-% vorliegt, wobei hierzu die Argon-Ion-Laservorrichtung eingesetzt wird.

Vorteilhaft ist es außerdem, daß in die Aufnahmevorrichtung zur Behandlung von Tumoren ein Festkörper oder gasförmiges oder flüssiges Gemisch einbringbar ist, das in der Konzentration von 10 x 10⁻⁶ Vol.-% bis 10 x 10⁻⁸ Vol.-% vorliegt, wobei hierzu die Argon-KryptonMischgas-Laservorrichtung eingesetzt wird.

Ferner ist es vorteilhaft, daß in die Aufnahmevorrichtung zur Behandlung von Gehirnerschütterungen ein Festkörper oder gasförmiges oder flüssiges Gemisch einbringbar ist, das in der Konzentration von 10 x 10⁻³ Vol.-% bis 10 x 10⁻⁹ Vol.-% vorliegt, wobei hierzu die Argon-Ion-Laservorrichtung eingesetzt wird.

Eine zusätzliche Möglichkeit ist, daß in die Aufnahmevorrichtung zur Behandlung von Stoffwechselerkrankungen ein Festkörper oder gasförmiges oder flüssiges Gemisch einbringbar ist, das in der Konzentration von 10 x 10⁻¹ Vol.-% bis 10 x 10⁻²⁴ Vol.-% vorliegt, wobei hierzu die Argon-Ion- und/oder Argon-Krypton-Mischgas- und/oder Dioden- und/oder Neodym-Yak und/oder He-Ne- und/oder He-Cd-Laservorrichtung eingesetzt wird.

Eine Anordnung zur Durchführung des erfindungsgemäßen Verfahrens ist, daß in die Aufnahmevorrichtung zur Behandlung von Zellkulturen ein Festkörper oder gasförmiges oder flüssiges Gemisch einbringbar ist, das in der Konzentration von 100 Vol.-% bis 10 x 10⁻²⁴ Vol.-% vorliegt, wobei hierzu die Argon-Ion-, Argon-Krypton-, Krypton-, Dioden-, Neodym-Yak-, He-Ne-, He-Cd-Laservorrichtung eingesetzt wird.

Die in dieser Beschreibung angegebenen Konzentrationen sind homogene Mischungen aus verschiedenen Stoffen. Für praktische Zwecke wird die Konzentration einer betrachteten Substanz bevorzugt in Gewichts- oder Volumenprozenten (Gewichts- oder Volumenanteil x 100 Vol.-%) angegeben. Für theoretische Überlegungen stellen der Molenbruch (Mole je Summe der Mole), die Molarität (Mole je Liter Lösung) oder die Molalität (Mole je kg Lösungsmittel) ein geeignetes Konzentrationsmaß dar.

**Weitere Vorteile und Einzelheiten der Erfindung sind in den Patentansprüchen** und in der Beschreibung erläutert und in den Figuren dargestellt.

Es zeigt:
- Figur 1: eine schematische Darstellung einer Laservorrichtung mit einem an der Auslaßöffnung der Laservorrichtung vorgesehenen Kabel bzw. Glasfaserkabel,
- Figur 2: eine Behandlungssonde bzw. eine Aufnahmevorrichtung, die über ein Kabel bzw. Glasfaserkabel mit der Laservorrichtung verbindbar ist.

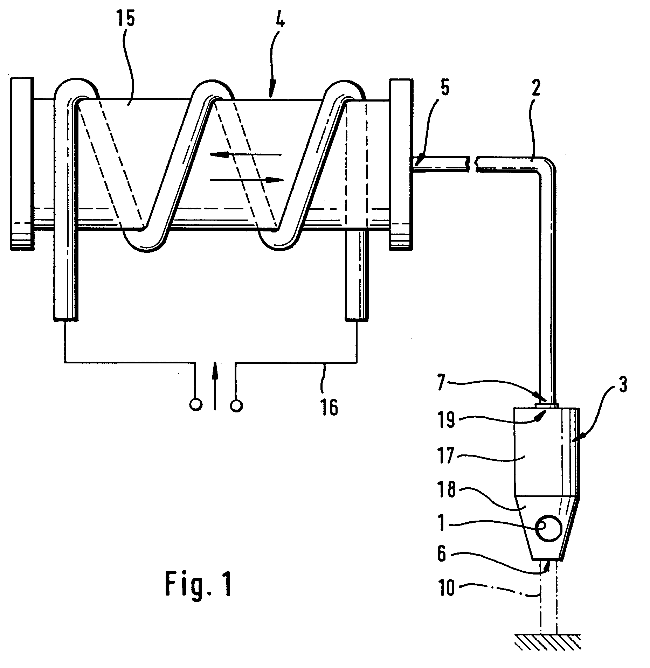

In der Zeichnung ist in Figur 1 eine Vorrichtung zur kohärenten Verstärkung elektromagnetischer Schwingungen mit 4 bezeichnet. Die Vorrichtung 4 besteht aus einem Laserrohr 15, in dem z. B. für Brandwunden ein Argon-Ion-Gas eingebracht ist. Über eine elektrische Leitung 16 wird der Laservorrichtung 4 Energie zugeführt. Die aus der Laservorrichtung 4 austretenden elektromagnetischen Schwingungen, Wellen bzw. der Laserstrahl können Frequenzen von 5 x 10¹⁴ Hz bis 10¹⁵ Hz (sichtbarer Bereich) aufweisen. Je nach Heilbehandlung bzw. Anwendungsart kann die Laservorrichtung so ausgelegt sein, daß sie im Ultraviolett- oder Infrarotbereich eingesetzt werden kann. Die hier angegebenen Frequenzen geben den sichtbaren Anteil (Licht) des elektromagnetischen Spektrums wieder.

Ein Laserstrahl bzw. ein Lichtstrahl 10 wird über ein Kabel 2 geleitet, dessen Einlaßöffnung 5 mit der Laservorrichtung 4 und dessen Auslaßöffnung und mit einer Einlaßöffnung 19 einer Behandlungssonde bzw. Aufnahmevorrichtung 3 in Verbindung steht. Über die Auslaßöffnung 6 trifft der Laserstrahl 10 nach außen und wird auf ein biologisches System 11 gerichtet.

Die Aufnahmevorrichtung 3 besteht aus einem zylinderförmigen Gehäuseteil 17 mit einem sich daran anschließenden, sich nach vorne verjüngenden Gehäuseteil 18. Über die gesamte Länge der Aufnahmevorrichtung 3 erstreckt sich eine Längsöffnung 9, die in die Auslaßöffnung 6 mündet. Über die Längsöffnung 9 wird also der Licht- bzw. Laserstrahl 10 geleitet, der dann über die in der Aufnahmevorrichtung 3 vorgesehene Auslaßöffnung 6 geführt wird, auf das biologische System 11 bzw. einen menschlichen Körper, einen Tierkörper oder einen pflanzlichen Körper auftrifft und dort die gewünschte Heilwirkung bzw. Veränderung im biologischen System herbeiführt. Im Bereich der Auslaßöffnung 6 der Aufnahmevorrichtung 3 befindet sich eine Queröffnung 1, die die Längsöffnung 9 in einem Winkel, in vorteilhafter Weise in einem rechten Winkel, schneidet.

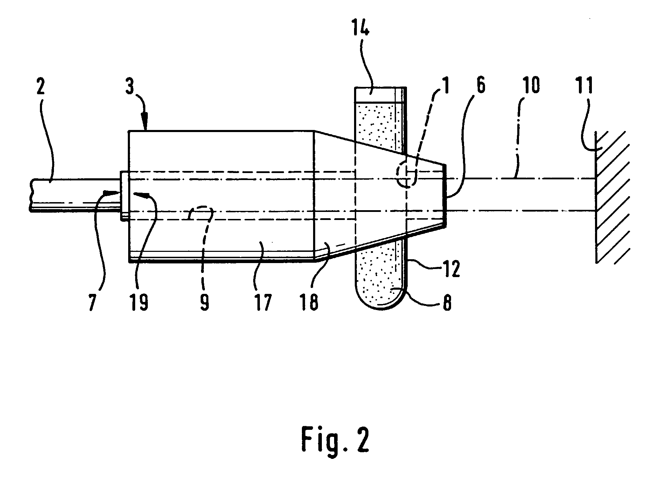

Wie aus Figur 2 hervorgeht, ist in die Queröffnung 1 ein Behälter 12 derart eingeführt, daß er den Strahlenkranz des Laserstrahls in einem Winkel von ca. 90° schneidet. Dadurch werden die Laserstrahlen über bzw. durch den Behälter 12 geleitet. Der Behälter 12 ist deshalb in vorteilhafter Weise aus Glas, insbesondere aus Quarzglas, gebildet, so daß fast keine Verluste auftreten, wenn der Laserstrahl das Quarzglas passiert.

Der Behälter 12 kann in vorteilhafter Weise zylinderförmig ausgebildet sein. Es ist jedoch auch möglich, den Behälter 12 konisch auszubilden und die entsprechende Queröffnung 1 ebenfalls der konischen Ausbildung des Behälters 12 anzupassen, so daß eine Klemmverbindung zwischen der Aufnahmevorrichtung 3 und dem Behäl-ter 12 erreicht wird.

Der Behälter 12 dient zur Aufnahme eines oder mehrerer Festkörper bzw. zur Aufnahme von Substanzen in pulvriger Form oder zur Aufnahme einer gasförmigen oder flüssigen Lösung. In den Behäter 12 kann zusätzlich eine Trägerlösung, z. B. Wasser und Äthanol im Verhältnis 1:1, eingefüllt werden. In die Trägerlösung werden die Beimischungen oder Substanzen in einer Konzentration von 50 Vol.-% bis zu 10 x 10⁻²⁴ Vol.-% in abnehmender Tendenz zugegeben. In diese Trägerlösung werden z. B. mineralische, pflanzliche, tierische oder menschliche Extrakte bzw. Produkte oder Giftstoffe zusätzlich eingegeben. Diese Zusatzstoffe bezeichnet man als Beimischung 8, die entscheidend für die positive Beeinflussung des biologischen Systems 11 oder die entsprechende medizinische Behandlung ist.

Die Befüllung des Behälters 12 kann also eine echte Lösung, ein Lösungsgemisch oder eine kolloidale Lösung sein. Je nach Zusammensetzung der Beimischung kann auch auf die Trägerlösung verzichtet werden.

Der Laserstrahl 10 wird über den Behälter 12 mit den Substanzen bzw. Beimischungen geleitet und trifft dann auf das biologische System 11. In jeder Atom- oder Molekülverbindung wird in vorteilhafter Weise die Lichtstrahlung spezifisch gestreut (Ausnutzung des Ramann-Effekts), und das austretende Licht enthält praktisch den Fingerabdruck der Beimischungen. Wird beispielsweise ein Argon-Ionen-Laser eingesetzt, liefert dieser Laserstrahl die entsprechende Grundenergie für die biologischen Prozesse. Der austretende Laserstrahl gibt dann die Informationen an das biologische System weiter und bewirkt den Heilungsprozeß z. B. bei einer Brandwunde.

Der Abstand zwischen dem Austrittsende der Aufnahmevorrichtung 3 und dem biologischen System 11 ist beim Behandlungsprozeß meist konstant. Vergrößert man den Abstand zwischen dem Austrittsende der Aufnahmevorrichtung 3 und dem biologischen System 11, so wird auch eine größere Fläche des biologischen Systems mittels des Laserstrahls 10 erreicht.

Wird beispielsweise bei der Wundbehandlung oder auch einer anderen Behandlungsart der Neodym-Yak-Laser eingesetzt, so ist es vorteilhaft, wenn die Wellenlänge für 532 nm (grün) und 473 nm (blau) verwendet wird.

Besonders vorteilhaft ist es, wenn die Laseremission im sichtbaren Bereich, also zwischen 650 nm und 300 nm (UV), liegt. Die Wahl der Wellenlänge hängt davon ab, welches biologische System 11 bestrahlt werden soll. Infrarotes Licht sollte nicht für menschliche Zellen verwendet werden, da es menschliche Zellen abtöten kann.

Wird beispielsweise die Laservorrichtung zur Tumorbehandlung eingesetzt, so besteht der Laser aus einem Argon-Ion-Krypton-Ion-Mischgaslaser zwischen 2 mm und 100 mm.

In diesem Fall werden folgende Beimischungen in den Behälter eingegeben:
a) Lokal: Extrakte aus Krankheitserregern von Tuberkulose und/oder Gonorrhoe und/oder Syphilis in Konzentration von 10 x 10⁻⁶ Vol.-% bis 10 x 10⁻⁸ Vol.-%.
b) Limbisches System (Gehirn): Auszug aus Salamandra 10 x 10⁻⁶ Vol.-% bis 10 x 10⁻⁸ Vol.-%.

Bei der Behandlung von Knochenbrüchen setzt man in vorteilhafter Weise einen Argon-Ion-Laser zwischen 2 mm und 100 mm ein.

Bei dieser Behandlung setzt man Substanzen als Beimischung ein, wie sie auf Seite 14 aufgelistet sind.

Aus vorstehenden Ausführungen ergibt sich, daß zur medizinischen Behandlung des biologischen Systems 11 und je nach Krankheitsart ein ganz bestimmter Lasertyp mit einer entsprechenden Sonde eingesetzt werden muß, wobei jeder Sonde bzw. Aufnahmevorrichtung eine bestimmte Beimischung 8 eingebracht wird.

Bei einem 1927 geborenen Patienten wurde eine offene, nichtheilende Wunde am linken Oberschenkel festgestellt. Diese Wunde ließ sich mit keiner klassischen Versorgung (z. B. Antibiotikagaben, chirurgische Wundrandbehandlung) positiv beeinflussen. Die Behandlung dieser Wunde erfolgte mit dem beschriebenen Argon-Ion-Laser, wobei die emittierten Frequenzen im sichtbaren Bereich lagen. Die Leistungsaufnahme der Laservorrichtung 4 lag bei 50 mW. Der Laserstrahl 10 wurde über die im Quarzglasbehälter 12 aufgenommenen Substanzen 8 auf die Brandwunde 11 geleitet. In der Trägerlösung waren mehrere Substanzen in folgender Konzentration enthalten: Auszug aus Aconitum 10 x 10⁻⁴ Vol.-% und Auszug aus Arnika 10 x 10⁻³ Vol.-%.

Der austretende Laserstrahl 10 wurde, wie bereits erwähnt, über den als optische Einrichtung wirkenden Behälter 12 auf die gesamte Wunde gestrahlt. Die Behandlungszeit betrug jeweils 30 Minuten. Schon nach einer Woche trat eine starke Heilreaktion ein. Die Wunde, die bei Beginn der Laserbestrahlung 10 cm lang und in der Mitte 2,5 cm breit, d. h. 25 cm² groß war, hatte nach einer Woche Behandlung eine Größe von 2 cm Durchmesser und nach zwei Wochen eine Größe von 7 mm Durchmesser. Nach drei Wochen war die Wunde vollständig abgeheilt.

Die Laserbestrahlung wurde erfolgreich bei Wundbehandlung, Knochenbrüchen, Zerrungen, Stauchungen und Gehirnerschütterungen eingesetzt.

Bei einer schweren Gehirnerschütterung und einer nach fünf Minuten aufgenommenen Laserbestrahlung konnte schon nach zehnminütiger Behandlung völlige Beschwerdefreiheit und normale neurologische Reaktion festgestellt werden.

Je nach Behandlungsart wird der entsprechende Laser mit dem zugehörigen, eine entsprechende Beimischung aufweisenden Behälter 12 eingesetzt.

Mit der Laserbehandlung bzw. Bestrahlung kann auch der Stoffwechsel positiv beeinflußt werden. Die entsprechenden Beimischungen enthalten Auszüge aus Pflanzenextrakten, aus tierischen und aus menschlichen Produkten, ferner Auszüge aus Viren, Bakterien, Pilzen und anderen Krankheitserregern sowie Auszüge aus Tumoren und pathologischen Sekreten.

Bei Organspenden können Zellkulturen und lebende Zellen positiv beeinflußt werden. Ferner ist es vorteilhaft, die Laserbestrahlung bei Mikroorganismen und auch Viren außerhalb von Lebewesen einzusetzen.

Nachstehende Lösungen wurden für die Wundbehandlung in folgender Konzentration erfolgreich eingesetzt:
- Auszug aus Aconitum: 10 x 10⁻⁴ Vol.-%
- Auszug aus Arnika: 10 x 10⁻³ Vol.-%
- Auszug aus Belladonna: 10 x 10⁻⁴ Vol.-%
- Auszug aus Bellis: 10 x 10⁻⁴ Vol.-%
- Auszug aus Calendula: 10 x 10⁻⁵ Vol.-%
- Auszug aus Chamomilla: 10 x 10⁻⁴ Vol.-%
- Auszug aus Echinacea ang.: 10 x 10⁻⁴ Vol.-%
- Auszug aus Echinacea purp.: 10 x 10⁻⁴ Vol.-%
- Auszug aus Hamamelis: 10 x 10⁻³ Vol.-%
- Auszug aus Millefolium: 10 x 10⁻⁴ Vol.-%
- Auszug aus Symphytum: 10 x 10⁻⁹ Vol.-%
- Lösung Hepar sulf.: 10 x 10⁻⁹ Vol.-%
- Lösung Merkurius sol.: 10 x 10⁻⁹ Vol.-%
- Lösung Quarz: 10 x 10⁻⁶ Vol.-%

Diese und andere Konzentrationen werden in den Behälter 12 eingegeben. Der Behälter 12 funktioniert als optischer Körper bzw. als Linse und weitet den Laserstrahl 10 auf. Beim Auftreffen des Laserstrahls auf ein Atom oder ein Molekül wird der Laserstrahl gestreut, d. h. es wird der erwähnte Ramann-Effekt genutzt. Der austretende Laserstrahl enthält den erwähnten optischen Fingerabdruck der vorstehenden Substanzen und nimmt Einfluß auf die Behandlung des biologischen Systems 11. Die Streustrahlen produzieren ein Biophotonenfeld.

Für die Behandlung von Knochenbrüchen werden Argon-Ion-Laser eingesetzt und Substanzen 8 in den Behälter 12 eingebracht. Diese Beimischung oder Substanz weist eine Konzentration von 10 x 10⁻³ bis 10 x 10⁻¹² Vol.-% auf.

Für die Behandlung der Wildrosen wird ein roter Diodenlaser 632 nm eingesetzt und ein Gemisch-Quarz in der Konzentration von 10 x 10⁻⁶ eingegeben.

Wird beispielsweise die Laservorrichtung zur Allergiebehandlung eingesetzt, so besteht der Laser aus einem roten He-Ne- oder Diodenlaser bei 632 nm und einem grünen He-Ne/Neodym-Yak-Laser.

In diesem Fall werden folgende Beimischungen in den Behälter gegeben: Auszug aus Dioscorea vilosa in der Konzentration von 10 x 10⁻⁵ Vol.-% und Kalium carb. in der Konzentration von 10 x 10⁻⁶ Vol.-%.

Zur Behandlung von Haarausfall besteht der Laser aus einem grünen He-Ne- oder Neodym-Yak-Laser.

In diesem Fall werden folgende Beimischungen in den Behälter gegeben: Extrakt aus Tuberkulose und/oder Placenta und/oder Extrakt aus Pel talpae in der Konzentration von 10 x 10⁻³ Vol.-% bis 10 x 10⁻⁹ Vol.-%

Wird die Laservorrichtung zur Behandlung von Darmentzündung eingesetzt, so besteht der Laser aus einem grünen He-Ne- oder Neodym-Yak-Laser im Wechsel mit einem gelben He-Ne-Laser.

In diesem Fall werden folgende Beimischungen in den Behälter gegeben: Extrakte aus Krankheitserregern in der Konzentration von 10 x 10⁻⁴ bis 10 x 10⁻¹² Vol.-%.

Wird beispielsweise die Laservorrichtung zur Behandlung von Durchblutungsstörungen eingesetzt, so besteht der Laser aus einem roten He-Ne-Diodenlaser.

In diesem Fall werden folgende Beimischungen in den Be-hälter gegeben: Extrakte aus Krankheitserregern und/oder Schlangengiften in der Konzentration von 10 x 10⁻⁴ Vol.-% bis 10 x 10⁻¹² Vol.-%.

Zur Behandlung von Ekzemen besteht der Laser aus einem grünen Laser im Wechsel mit einem Argon-Ion-Multiline-Laser.

In diesem Fall werden folgende Beimischungen in den Behälter gegeben: Extrakte aus Krankheitserregern in der Konzentration von 10 x 10⁻⁴ bis 10 x 10⁻¹² Vol.-%.

Für die Behandlung von Abwehrschwäche (Immunschwäche) besteht der Laser aus einem roten He-Ne-Diodenlaser.

In diesem Fall werden folgende Beimischungen in den Behälter gegeben: Extrakte aus Krankheitserregern in der Konzentration von 10 x 10⁻⁴ bis 10 x 10⁻¹² Vol.-%.

Zur Behandlung einer Gallenkolik besteht der Laser aus einem grünen He-Ne- oder Neodym-Yak-Laser.

In diesem Fall werden folgende Beimischungen in den Behälter gegeben: Extrakte aus Krankheitserregern, Magnesium phosphat und Calcium phosphat in der Konzentration von 10 x 10⁻⁴ Vol.-% bis 10 x 10⁻¹² Vol.-%.

Wird beispielsweise die Laservorrichtung zur Behandlung von Magenbeschwerden eingesetzt, so besteht der Laser aus einem roten He-Ne-Diodenlaser.

In diesem Fall werden folgende Beimischungen in den Behälter gegeben: Extrakte aus Krankheitserregern in der Konzentration von 10 x 10⁻⁴ bis 10 x 10⁻¹² Vol.-%.

Wird die Laservorrichtung zur Behandlung von Gelenkbeschwerden eingesetzt, so besteht der Laser aus einem Argon-Ion-Multiline-Laser.

In diesem Fall werden folgende Beimischungen in den Behälter gegeben: Extrakte aus Krankheitserregern in der Konzentration von 10 x 10⁻⁴ bis 10 x 10⁻¹² Vol.-%.

Zur Behandlung von Verdauungsorganen und der Nieren besteht der Laser aus einem gelben und einem grünen He-Ne-Laser.

In diesem Fall werden folgende Beimischungen in den Behälter gegeben: Extrakte aus Krankheitserregern in der Konzentration von 10 x 10⁻⁴ bis 10 x 10⁻¹² Vol.-% und/oder Mineralien in der Konzentration von 10 x 10⁻³ Vol.-% bis 10 x 10⁻²⁴ Vol.-% und/oder Extrakt aus Salamandra in der Konzentration von 10 x 10⁻⁸ Vol.-%.

Wird die Laservorrichtung zur Behandlung von Herpes und Herpes zoster eingesetzt, so besteht der Laser aus einem Argon-Ion-Multiline-Laser.

In diesem Fall werden folgende Beimischungen in den Behälter gegeben: Extrakt aus Tuberkulose in der Konzentration von 10 x 10⁻⁶ Vol.-%.

Für die Behandlung von Nervenentzündungen (Ischialgie) besteht der Laser aus einem Argon-Ion-Multiline-Laser.

In diesem Fall werden folgende Beimischungen in den Behälter gegeben: Extrakt aus Tuberkulose in der Konzentration von 10 x 10⁻⁶ Vol.-%.

Zur Behandlung von Herzschwäche, pektanginöse Beschwerden, besteht der Laser aus einem orangefarbenen He-Ne-Laser.

In diesem Fall werden folgende Beimischungen in den Behälter gegeben: Extrakte aus Krankheitserregern in der Konzentration von 10 x 10⁻⁴ bis 10 x 10⁻¹² Vol.-% und/oder Mineralien in der Konzentration von 10 x 10⁻³ bis 10 x 10⁻²⁴ Vol.-% und/oder Extrakt aus Salamandra in der Konzentration von 10 x 10⁻⁸ Vol.-%.

Wird beispielsweise die Laservorrichtung zur Behandlung von Migräne eingesetzt, so besteht der Laser aus einem Argon-Ion-Multiline-Laser und einem roten He-Ne-Dioden-Laser.

In diesem Fall werden folgende Beimischungen in den Behälter gegeben: Extrakt aus Tuberkulose in der Konzentration von 10 x 10⁻⁶ Vol.-%.

Zur Behandlung von Neurodermitis besteht der Laser aus einem Argon-Krypton-Mischgaslaser und einem roten He-Ne-Dioden-Laser.

In diesem Fall werden folgende Beimischungen in den Behälter gegeben: Extrakte aus Krankheitserregern in der Konzentration von 10 x 10⁻⁴ bis 10 x 10⁻¹² Vol.-% und/oder Mineralien in der Konzentration von 10 x 10⁻³ bis 10 x 10⁻²⁴ Vol.-% und/oder Extrakt aus Salamandra in der Konzentration von 10 x 10⁻⁸ Vol.-% und einem Auszug aus Dioscorea vilosa in der Konzentration von 10 x 10⁻⁵ Vol.-% und Kalium carb. in der Konzentration von 10 x 10⁻⁶ Vol.-%.

Wird die Laservorrichtung zur Behandlung von Parkinson eingesetzt, so besteht der Laser aus einem Argon-Ion-Multiline-Laser und einem Argon-Krypton-Mischgaslaser.

In diesem Fall werden folgende Beimischungen in den Behälter gegeben: Extrakte aus Krankheitserregern in der Konzentration von 10 x 10⁻⁴ bis 10 x 10⁻¹² Vol.-% und Extrakte aus Hormonen in der Konzentration von 10 x 10⁻⁶ bis 10 x 10⁻²⁴ Vol.-%.

Für die Behandlung von Drüsenstörungen besteht der Laser aus einem Argon-Ion-Multiline-Laser, einem ArgonKrypton-Mischgaslaser und einem grünen He-Ne- oder Neodym-Yak-Laser.

In diesem Fall werden folgende Beimischungen in den Behälter gegeben: Extrakte aus Krankheitserregern in der Konzentration von 10 x 10⁻⁴ bis 10 x 10⁻¹² Vol.-% und Extrakte aus Hormonen in der Konzentration von 10 x 10⁻⁶ bis 10 x 10⁻²⁴ Vol.-%.

### Bezugszeichenliste

- 1: Queröffnung
- 2: Kabel, Glasfaserkabel
- 3: Behandlungssonde, Aufnahmevorrichtung, Sonde
- 4: Vorrichtung zur kohärenten Verstärkung elektromagnetischer Schwingungen (Laservorrichtung) = Laser
- 5: Einlaßöffnung des Kabels 2
- 6: Auslaßöffnung der Vorrichtung 3
- 7: Auslaßöffnung des Kabels 2
- 8: Beimischung; Festkörper, Gemisch, flüssige oder gasförmige (Substanz) Lösung
- 9: Längsöffnung
- 10: elektromagnetische Schwingungen, Wellen, Laserstrahl bzw. Strahleingang des Laserstrahls
- 11: biologisches System, menschlicher Körper, Tierkörver (Brandwunde)
- 12: Behälter
- 14: Verschlußeinrichtung
- 15: Laserrohr
- 16: elektrische Leitung
- 17: Gehäuseteil der Aufnahmevorrichtung 3
- 18: Gehäuseteil der Aufnahmevorrichtung 3
- 19: Einlaßöffnung der Aufnahmevorrichtung 3

## Patentansprüche

1. Vorrichtung (4) zur Verstärkung elektromagnetischer Schwingungen durch induzierte Emission mittels eines Festkörpers, mit Neodym dotierte Kristalle oder Gläser, Halbleiter-Dioden oder Flüssigkeiten oder Gase, wobei die aus der Vorrichtung austretenden elektromagnetischen Schwingungen über ein Kabel oder Glasfaserkabel (2) zur Beeinflussung auf ein biologisches System, insbesondere auf einen menschlichen oder Tierkörper (11), geleitet werden, **dadurch gekennzeichnet**, daß das Kabel (2) eine Auslaßöffnung (7) aufweist und die aus dem Kabel (2) austretenden Schwingungen oder Strahlen über einen Festkörper, ein Gemisch oder eine flüssige oder gasförmige Lösung (8) zur Beeinflussung auf das biologische System (11) geleitet werden, wobei der Festkörper, das Gemisch, die flüssigen oder die gasförmigen Lösungen mineralische, pflanzliche, tierische, menschliche Extrakte bzw. Produkte oder Giftstoffe als Beimischungen enthalten.

2. Vorrichtung nach Anspruch 1, **dadurch gekennzeichnet**, daß das Kabel (2) eine Einlaßöffnung (5) und die Auslaßöffnung (7) aufweist, wobei die Auslaßöffnung (7) in die Einlaßöffnung (19) einer Behandlungssonde (3) einmündet und zwischen den Ein- und Auslaßöffnungen (19, 6) der Festkörper, das Gemisch oder die gasförmige oder flüssige Lösung (8) vorgesehen bzw. einbringbar ist.

3. Vorrichtung nach Anspruch 1, **dadurch gekennzeichnet**, daß die Behandlungssonde oder die Aufnahmevorrichtung (3) eine Längsöffnung (9) und eine die Längsöffnung (9) in einem Winkel schneidende Queröffnung (1) aufweist, in die der Festkörper, das Gemisch oder die gasförmige oder flüssige Lösung (8) mittel- oder unmittelbar einbringbar ist.

4. Vorrichtung nach Anspruch 1 und 3, **dadurch gekennzeichnet**, daß die Queröffnung (1) in einem Strahleingang (10) der elektromagnetischen Schwingungen bzw. des Laserstrahls liegt und zur Aufnahme eines Behälters (12) dient, der zur Aufnahme des Festkörpers, des Gemischs oder der gasförmigen oder flüssigen Lösung (8) dient.

5. Vorrichtung nach Anspruch 1 und 4, **dadurch gekennzeichnet**, daß der Behälter (12) zur Aufnahme des Festkörpers, der gasförmigen oder flüssigen Substanz oder des Gemischs (8) aus einem Glas oder einem Quarzglas besteht.

6. Vorrichtung nach Anspruch 1 und 5, **dadurch gekennzeichnet**, daß der Behälter (12) in die Queröffnung (1) einsetzbar und festklemmbar und/oder arretierbar ist.

7. Vorrichtung nach den Ansprüchen 1 bis 6, **dadurch gekennzeichnet**, daß der Behälter (12) mit einer Verschlußeinrichtung (14) versehen ist.

8. Vorrichtung nach Anspruch 1, **dadurch gekennzeichnet**, daß die Vorrichtung oder die Laservorrichtung (4) als Argon-Ion-Laser und/oder als Argon-Ion-Multiline-Laser für Frequenzen von 5 x 10¹⁴ Hz bis 5 x 10¹⁵ Hz ausgelegt ist.

9. Vorrichtung nach Anspruch 1, **dadurch gekennzeichnet**, daß die in den Behälter (12) eingegebene Beimischung in eine Trägerlösung aus Wasser und Aethanol eingegeben ist, wobei die Beimischung der Substanzen in einer Konzentration von 90 Vol.-% bis 10 x 10⁻²⁴ Vol.-% vorliegt.

10. Vorrichtung nach Anspruch 1, **dadurch gekennzeichnet**, daß die Laservorrichtung eine Leistungsaufnahme zwischen 30 mW und 100 mW oder 40 mW und 70 mW oder 45 mW und 65 mW oder 45 mW und 55 mW, insbesondere 50 mW, hat

11. Vorrichtung nach Anspruch 10, **dadurch gekennzeichnet**, daß der Laser (4) als Argon-Ion-Krypton-Mischgas-Laser ausgelegt ist.

12. Vorrichtung nach einem der vorhergehenden Ansprüche, **dadurch gekennzeichnet**, daß der Laser (4) als Ne-Ne-Laser ausgelegt ist.

13. Vorrichtung nach einem der vorhergehenden Ansprüche, **dadurch gekennzeichnet**, daß der Laser (4) als He-Cd-Laser ausgebildet ist.

14. Vorrichtung nach einem der vorhergehenden Ansprüche, **dadurch gekennzeichnet**, daß der Laser (4) als Multiline-Singleline-, Multimod- oder als durchstimmbarer Laser ausgebildet ist.

15. Vorrichtung nach einem der vorhergehenden Ansprüche, **dadurch gekennzeichnet**, daß der Laser (4) als Dioden-Laser für Frequenzen von 5 x 10¹⁴ Hz bis 10¹⁵ Hz ausgelegt ist.

16. Vorrichtung nach Anspruch 15, **dadurch gekennzeichnet**, daß der Laser (4) als Kristall-Laser bzw. als Neodym-Yak-Laser ausgelegt ist.

17. Vorrichtung nach Anspruch 1, **dadurch gekennzeichnet**, daß in die Aufnahmevorrichtung (3) zur Behandlung von Prellungen ein Festkörper oder gasförmiges oder flüssiges Gemisch (8) einbringbar ist, das in der Konzentration von 10 x 10⁻³ bis 10 x 10⁻⁹ Vol.-% vorliegt.

18. Vorrichtung nach Anspruch 17, **dadurch gekennzeichnet**, daß in die Aufnahmevorrichtung (3) zur Behandlung von Knochenbrüchen ein Festkörper oder gasförmiges oder flüssiges Gemisch (8) einbringbar ist, das in der Konzentration von 10 x 10⁻³ bis 10 x 10⁻¹² Vol.-% vorliegt, wobei hierzu die Argon-Ion-Laservorrichtung (4) eingesetzt wird.

19. Vorrichtung nach Anspruch 18, **dadurch gekennzeichnet**, daß in die Aufnahmevorrichtung (3) zur Behandlung von Tumoren ein Festkörper oder gasförmiges oder flüssiges Gemisch (8) einbringbar ist, das in der Konzentration von 10 x 10⁻⁶ bis 10 x 10⁻⁸ Vol.-% vorliegt, wobei hierzu die Argon-Krypton-Mischgas-Laservorrichtung (4) eingesetzt wird.

20. Vorrichtung nach einem der vorhergehenden Ansprüche, **dadurch gekennzeichnet**, daß in die Aufnahmevorrichtung (3) zur Behandlung von Gehirnerschütterungen ein Festkörper oder gasförmiges oder flüssiges Gemisch (8) einbringbar ist, das in der Konzentration von 10 x 10⁻³ bis 10 x 10⁻⁹ Vol.-% vorliegt, wobei hierzu die Argon-Ion-Laservorrichtung (4) eingesetzt wird.

21. Vorrichtung nach einem der vorhergehenden Ansprüche, **dadurch gekennzeichnet**, daß in die Aufnahmevorrichtung (3) zur Behandlung von Stoffwechselerkrankungen ein Festkörper oder gasförmiges oder flüssiges Gemisch (8) einbringbar ist, das in der Konzentration von 10 x 10⁻¹ bis 10 x 10⁻²⁴ Vol.-% vorliegt, wobei hierzu die Argon-Ion- und/oder Argon-Krypton-Mischgas- und/oder Dioden- und/oder Neodym-Yak- und/oder He-Ne- und/oder He-Cd-Laservorrichtung (4) eingesetzt wird.

22. Vorrichtung nach einem der vorhergehenden Ansprüche, **dadurch gekennzeichnet**, daß in die Aufnahmevorrichtung (3) zur Behandlung von Zellkulturen ein Festkörper oder gasförmiges oder flüssiges Gemisch (8) einbringbar ist, das in der Konzentration von 100 Vol.-% bis 10 x 10⁻²⁴ Vol.-% vorliegt, wobei hierzu die Argon-Ion-, Argon-Krypton-, Krypton-, Dioden-, Neodym-Yak-, He-Ne-, He-Cd-Laservorrichtung (4) eingesetzt wird.
